# EUROPEAN PATENT APPLICATION

(11) **EP 4 488 301 A1**
(43) Date of publication of application: **08.01.2025**
(21) Application number: 23763322.7
(22) Date of filing: 22.02.2023
(51) Int. Cl.: C08F 20/36, C09K 15/02

(54) **AMIDO GROUP-CONTAINING MONOMER COMPOSITION AND METHOD FOR IMPROVING STORAGE STABILITY OF AMIDO GROUP-CONTAINING MONOMER**

(30) Priority: 01.03.2022 JP 2022030805
(71) Applicant: NOF Corporation, Shibuya-ku Tokyo 150-6019 (JP)
(72) Inventor: KATOH Masanori, Amagasaki-shi Hyogo 660-0095 (JP); TAGAMI Yasunobu, Amagasaki-shi Hyogo 660-0095 (JP); AONO Tatsuya, Amagasaki-shi Hyogo 660-0095 (JP); MATSUMOTO Shinnosuke, Amagasaki-shi Hyogo 660-0095 (JP)
(74) Representative: Mewburn Ellis LLP
(86) International application number: PCT/JP2023/006354
(87) International publication number: WO 2023/167063

(57) **Abstract**

(Object) It is to provide an amido group-containing monomer composition in which the thickening due to generation of self-polymerization product and the precipitation and coloration after forming a coating liquid are reduced even after the storage at a high temperature.

(Solution) An amido group-containing monomer composition contains 80.00 to 99.99 mass% of an amido group-containing monomer (a) of a formula (1) and 0.01 to 20.00 mass% of a metal chelate complex (b) having carbonyl group as a ligand. 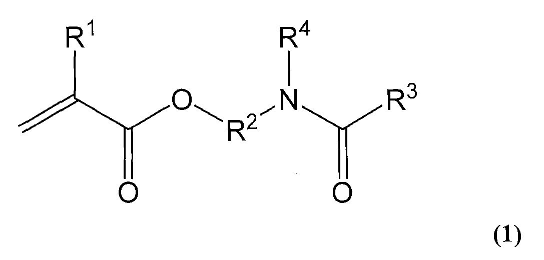 (R¹ represents hydrogen atom or methyl group, R² represents a hydrocarbon group having a carbon number of 1 to 6, R³ represents a hydrocarbon group having a carbon number of 1 to 23, and R⁴ represents hydrogen or methyl group.)

## Description

### TECHNICAL FIELD

The present invention is related to a specific amido group-containing monomer composition and a method of stabilizing a specific amido-group containing monomer.

### BACKGROUND ARTS

An amido group-containing monomer such as alkanol amido (meth) acrylate has high compatibility with a monomer or an organic solvent and hydrophilic property or hydrophobic property can be imparted by adjusting the length of the alkyl chain. Thus, copolymers of the amido group-containing monomers have been applied in applications such as a fiber reforming agent (Patent document 1: Japanese patent publication No. 2020-128529A), an active energy ray-curable ink (Patent document 2: Japanese patent publication No. 6775758B and Patent document 3: Japanese patent publication No. 2017-160380A) and the like.

Further, the amido group-containing monomer such as alkanol amido (meth) acrylate is problematic in the thickening and gelation due to self-polymerization during storage at a high temperature. For example, the temperature may exceed 40°C depending on the storage condition during transportation in summer, and the thickening, gelation or insoluble content occurs at this time.

As a general method for preventing the self-polymerization of a (meth)acrylate monomer, it is listed to add a polymerization inhibitor, including a phenol-based compound such as hydroquinone (Patent document 4: Japanese patent publication No. 1979 (S54) -014904A), a nitroso compound (Patent document 5: Japanese examined patent publication No. 1983 (S58)-046496B) or a phenothiazine-based compound (Patent document 6: Japanese patent publication No. 1996 (H8)-040979).

### [PRIOR TECHNICAL DOCUMENTS]

### [PATENT DOCUMENTS]

[PATENT DOCUMENT 1] Japanese patent publication No. 2020-128529A
[PATENT DOCUMENT 2] Japanese patent No. 6775758B
[PATENT DOCUMENT 3] Japanese patent publication No. 2017-160380A
[PATENT DOCUMENT 4] Japanese patent publication No. 1979 (S54)-014904A
[PATENT DOCUMENT 5] Japanese examined patent No. 1983 (S58)-046496B
[PATENT DOCUMENT 6] Japanese patent publication No. 1996 (H8)-040979A

### SUMMARY OF THE INVENTION

### (Object to be solved by the Invention)

However, in the case that various kinds of polymerization inhibitors described above are added to the amido-group-containing monomer such as alkanol amido (meth)acrylate, the effect of suppressing the polymerization is insufficient when the added amount is low. On the other hand, when the added amount of the polymerization inhibitor is high, coloration occurs to contribute to the coloration of a product.

An object of the present invention is to provide an amido group-containing monomer composition capable of suppressing the thickening due to the generation of a self-polymerization product and precipitation and coloration after producing a coating liquid, after the storage at a high temperature.

### (Solution for the Object)

As the inventors variously considered and studied the object described above and have found that the object described above can be solved by blending a specific metal chelate complex into a specific amido group-containing monomer.

That is, the present invention is defined as follows.
(1) An amido group-containing monomer composition comprising 80.00 to 99.99 mass% of an amido group-containing monomer (a) represented by a following formula (1), and 0.01 to 20.00 mass% of a metal chelate complex (b) having carbonyl group as a ligand. 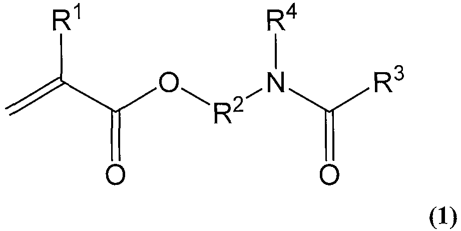 (In the formula (1),
   R¹ represents hydrogen atom or methyl group,
   R² represents a hydrocarbon group having a carbon number of 1 to 6,
   R³ represents a hydrocarbon group having a carbon number of 1 to 23, and
   R⁴ represents hydrogen or methyl group.)
(2) The amido group-containing monomer composition of (1), wherein said metal chelate complex (b) is represented by a following formula (2). 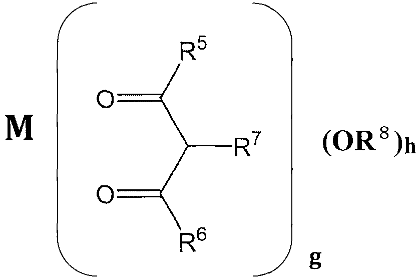 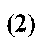 (In the formula (2),
   M represents a transition metal,
   g represents 1 to 4,
   h represents 0 to 3,
   g+h represents a total coordination number of said transition metal,
   R⁵ and R⁶ independently represent a hydrocarbon group having a carbon number of 1 to 5 or phenyl group, respectively,
   R⁷ represents hydrogen or a hydrocarbon group having a carbon number of 1 to 3, and
   R⁸ represents hydrogen or a hydrocarbon group having a carbon number of 1 to 6.) (3) A method of improving stability of storage of an amido group-containing monomer, said method comprising the step of adding a metal chelate complex (b) represented by a following formula (2) to an amido group-containing monomer (a) represented by a following formula (1). 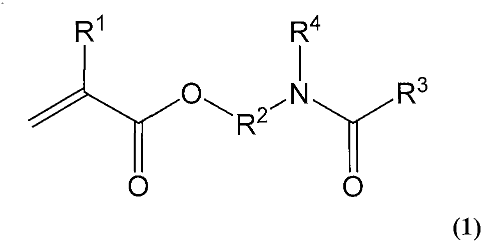 (In the formula (1),
      R¹ represents hydrogen atom or methyl group,
      R² represents a hydrocarbon group having a carbon number of 1 to 6,
      R³ represents a hydrocarbon group having a carbon number of 1 to 23, and
      R⁴ represents hydrogen or methyl group.) 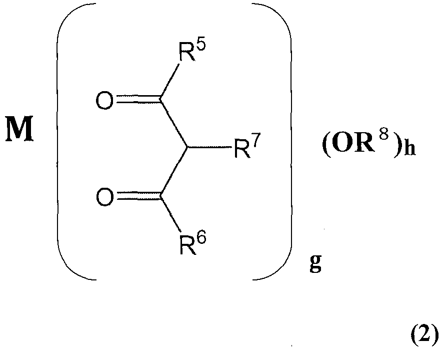 (In the formula (2),
         M represents a transition metal,
         g represents 1 to 4,
         h represents 0 to 3,
         g+h represents a total coordination number of said transition metal,
         R⁵ and R⁶ independently represent a hydrocarbon group having a carbon number of 1 to 5 or phenyl group, respectively,
         R⁷ represents hydrogen or a hydrocarbon group having a carbon number of 1 to 3, and
         R⁸ represents hydrogen or a hydrocarbon group having a carbon number of 1 to 6.)

### (Effect of the Invention)

According to the present invention, it is possible to provide an amido group-containing monomer composition capable of preventing the self-polymerization and coloration and excellent in stability during storage at a high temperature.

### EMBODIMENTS FOR CARRYING OUT THE INVENTION

Although embodiments of the present invention will be described below, the present invention is not limited to them.

The composition of the present invention contains 80.00 to 99.99 mass% of an amido group-containing monomer (a) represented by the formula (1) described above and 0.01 to 20.00 mass% of a metal chelate complex (b) having carbonyl group as a ligand. The respective components will be described below.

### (Amido group-containing monomer (a))

The amido group-containing monomer of the present invention is represented by the following formula (1).

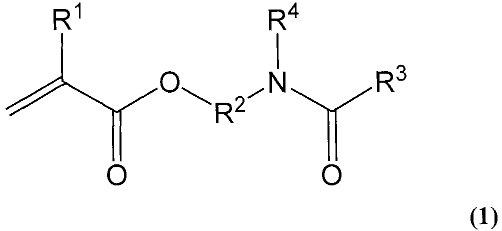

In the formula (1), R¹ represents hydrogen atom or methyl group.
R² represents a hydrocarbon group having a carbon number of 1 to 6. R² may be a straight-chain hydrocarbon group, a branched-chain hydrocarbon group, a saturated hydrocarbon group or an unsaturated hydrocarbon group. The carbon number of R² may more preferably be 1 to 4. R² may most preferably be alkylene group. On the viewpoint of availability, methylene group, ethylene group, trimethylene group and tetramethylene group are more preferred.
R³ is a hydrocarbon group having a carbon number of 1 to 23. R³ may be a straight-chain hydrocarbon group, a branched-chain hydrocarbon group, a saturated hydrocarbon group or an unsaturated hydrocarbon group. The carbon number of R³ may preferably be 1 to 21 and more preferably be 1 to 18. Preferably, R³ may be a straight-chain alkyl group or a branched-chain alkyl group, and a readily available straight-chain alkyl group having a carbon number of 1 to 21 is most preferred. For example, methyl group, ethyl group, propyl group, heptyl group, dodecyl group, tetradecyl group, pentadecyl group, heptadecyl group, nonadecyl group and eicosyl group are listed.
R⁴ is hydrogen or methyl group, and hydrogen is more preferred on the viewpoint of availability.

The amido group-containing monomer (a) may be applied alone or the two or more kinds may be applied in combination.

### (Metal chelate complex (b) having carbonyl group as a ligand)

The metal chelate complex (b) having carbonyl group as a ligand of the present invention is represented by the following formula (2).

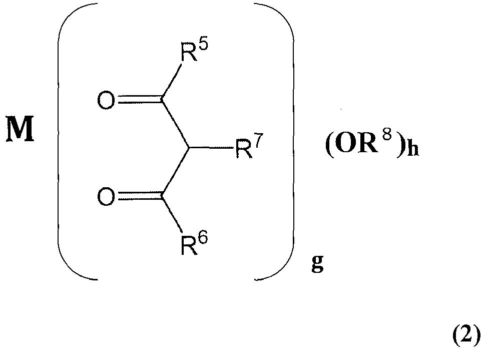

In the formula (2), M represents a transition metal, and may preferably be selected from aluminum, chromium, cobalt, vanadium, iron, zinc, zirconium, indium and titanium. On the viewpoint of suppressing self-polymerization upon storage, iron and zirconium are preferred and zirconium is more preferred due to the resistance against coloration.

g represents a coordination number of diketone, g is 1 to 4, h is 0 to 3, and g+h represents a total coordination number of a metal, g is preferably 3 to 4 on the viewpoint of preventing self-polymerization, and g is preferably 1 to 2 on the viewpoint of preventing the reduction of the purity upon storage.

R⁵ and R⁶ independently represent a hydrocarbon group having a carbon number of 1 to 5 or phenyl group, respectively. The hydrocarbon group may be a straight-chain hydrocarbon group, a branched-chain hydrocarbon group, a saturated hydrocarbon group or an unsaturated hydrocarbon group. The hydrocarbon group may be alkyl group and preferably be methyl group, ethyl group, normal propyl group, isopropyl group, butyl group or pentyl group, and methyl group is more preferred on the viewpoint of availability.

R⁷ represents hydrogen or a hydrocarbon group having a carbon number of 1 to 3. R⁷ may be a straight-chain hydrocarbon group, a branched-chain hydrocarbon group, a saturated hydrocarbon group or an unsaturated hydrocarbon group. The hydrocarbon group may preferably be alkyl group and more preferably be methyl group, ethyl group, n-propyl group, isopropyl group or the like, and hydrogen is most preferred on the viewpoint of availability.

R⁸ is hydrogen or a hydrocarbon group having a carbon number of 1 to 6. R⁸ may be a straight-chain hydrocarbon group, a branched-chain hydrocarbon group, a saturated hydrocarbon group or an unsaturated hydrocarbon group. The hydrocarbon group may preferably be an alkyl group and more preferably be methyl group, ethyl group, normal propyl group, isopropyl group, butyl group, pentyl group or hexyl group, and hydrogen is more preferred on the viewpoint of suppressing the reduction of the purity of a composition.

The metal chelate complex (b) may be applied alone or the two or more kinds may be applied in combination.

Provided that the total amount of the amido-group containing monomer (a) and metal chelate complex (b) is defined as 100.00 mass%, the concentration of the amido group-containing monomer (a) is made 80.00 to 99.99 mass%. In the case that the ratio of the amido group-containing monomer (a) is too high, the effect of preventing the self-polymerization cannot be obtained. The ratio is thus made 99.99 mass% or lower, and 99.95 mass% or lower is preferred and 99.90 mass% or lower is more preferred. Further, in the case that the ratio of the amido group-containing monomer (a) is too low, the metal chelate complex (b) may be precipitated. The ratio is thus made 80.00 mass% or higher, and 89.00 mass% or higher is preferred and 90.00 mass% or higher is more preferred.

### (Polymerization inhibitor)

A polymerization inhibitor may be optionally added to the composition of the present invention. As such component, although it is not limited, known compounds are listed as the polymerization inhibitor.

For example, polymerization inhibitors such as a phenol-based compound, a nitroso compound or a phenothiazine-based compound are listed.

The blending ratio of the polymerization inhibitor may preferably be 0 to 1.0 mass% and more preferably be 0.01 to 0.10 mass%, provided that the total amount of the amido group-containing monomer (a) and metal chelate complex (b) is defined as 100 mass%. In the case that the amount of the polymerization inhibitor is low, the effect of preventing the self-polymerization may be insufficient. In the case that the amount of the polymerization inhibitor is high, it may be the cause of the coloration.

### (Method of producing the composition)

The method of producing the composition may be performed as follows.
(1) The amido group-containing monomer (a) and metal chelate complex(b) are blended at a predetermined ratio and agitated so that the metal chelate complex is dissolved to produce the composition.
(2) When a (meth) acrylic acid and alkanol amido are subjected to esterification with dehydration under the presence of an acid catalyst to generate the amido group-containing monomer (a), the metal chelate complex (b) is added to the reaction system to produce the composition.
(3) When a (meth)acrylic ester and alkanol amido are subjected to transesterification reaction under the presence of a transesterification catalyst to generate the amido group-containing monomer (a), the metal chelate complex (b) is added to the reaction system to produce the composition.
(4) When acrylic chloride and alkanol amide are reacted to generate the amido group-containing monomer (a), the metal chelate (b) is added to the reaction system to produce the composition.

Further, as the metal chelate complex (b) itself of the present invention, having carbonyl group as a ligand, functions as a transesterification catalyst, the metal chelate complex can be applied as the transesterification catalyst in the transesterification reaction of the (meth)acrylic ester and alkanol amido described above. In the case that it is applied for the transesterification reaction, the activity may be increased. It is thus possible to suppress the denaturing of the amido group-containing monomer (a) by denaturing a part of carbonyl group into hydroxy group by adding a small amount of water.

The amido group-containing monomer (a) of the present invention may be applied by blending it into a paint or coating material as such, for example, or may be applied by polymerization through radical polymerization or the like. As the amido group of the amido group-containing monomer (a) has high aggregating property, it can be adhered to a substrate of a high polarity, so that it is possible to improve the adherence of a paint. Further, according to the polymer containing the amido group-containing monomer (a), it is possible to improve the glass transition temperature of the polymer due to the strong intermolecular interaction derived from the amido group.

In the case that the self-polymerization of the amido group-containing monomer (a) takes place, the self-polymerization product other than the target product is contained in the polymer produced by applying the amido group-containing monomer (a) as the copolymer, so that the physical property of the product is considerably deteriorated. Further, as the viscosity of the polymerization product of the amido group-containing monomer (a) is relatively high, the viscosity of the product applying the amido group-containing monomer (a) may be increased and precipitation may occur in the product. Thus, it is preferred that the self-polymerization product is not contained. Further, in the case that the amido group-containing monomer is colorized due to the self-polymerization and it is applied in a coating or paint, there is the problem that the coloration derived from the amido group-containing monomer (a) may deteriorate the color phase or design of the product.

The properties described above (viscosity, precipitation and coloration) due to the self-polymerization of the amido group-containing monomer (a) can be evaluated according to the method described below.

### EXAMPLES

### (Inventive examples and comparative examples)

Although embodiments of the present invention will be described further in detail below referring to the inventive examples and comparative examples, the present invention is not limited to them.

### (Evaluation of ratio of increase of viscosity)

A digital viscometer "DV-I Prime" manufactured by BROOKFIELD was applied to measure a viscosity (mPa·S) at a melting temperature at 70°C and a spindle of LV-1. The ratio of the increase of the viscosities before and after the storage test was calculated based on the following formula. Ratio of increase of viscosity (%) = [(viscosity after storage - viscosity before storage)/viscosity before storage] ×100 Judgement:
O: less than 20%
×: 20% or more

### (Evaluation of Gardner color number)

A sample was adjusted at 70°C and measured by Gardner color number meter.

### Judgement of Gardner color number

O: less than 7
×: 7 or more

### (Evaluation of solubility test)

10g of DMF was added to 0.5g of the sample and then agitated at 25°C for 30 minutes, and the appearance was observed by eyes.
O: Turbidity is not observed.
×: Turbidity is observed.

### (Evaluation of ratio of change of purity)

A gas chromatograph (Model: "GC-2014" produced by SHIMADZU CORPORATION) and a column "DB-1" were applied, and biphenyl was applied as an internal standard substance to measure the purity of the target substance in the sample, so that the ratio of change of purities before and after the long-term storage at 50°C. Ratio of change of purity (%) = [Purity (%) after storage/Purity (%) before storage] × 100

### Judgement of ratio of change of purity

O: 96% or higher
×: lower than 96%

### (Storage test at 50°C)

100g of each of the respective compositions of the inventive examples 1 to 12 and comparative examples 1 to 8 was weighed, left to stand at 50°C for 48 hours, and the ratio of the increase of the viscosity, Gardner color number, solubility test and ratio of the change of purity were measured as described above.

### (Long-term storage test at 50°C)

100g of each of the respective compositions of the inventive examples 1 to 12 and comparative examples 1 to 8 was weighed, left to stand at 50°C for 240 hours, and the ratio of the increase of the viscosity, Gardner color number, solubility test and ratio of the change of purity were measured as described above.

Table 1 indicates the structure (R¹, R², R³ and R⁴ in the formula (1)) of the amido group-containing monomer (a) and abbreviations.

Table 2 shows the structure (R⁵, R⁶, R⁷, R⁸, g and h in the formula (2)) of the metal chelate complex (b) or (b') and abbreviations.

Further, table 3 shows the polymerization inhibitors and abbreviations.

**Table 1**

| **Table 1, Amido group-containing monomer (a)** | | | | |
|---|---|---|---|---|
| | **R¹** | **R²** | **R³** | **R⁴** |
| **(a)-1** | **H** | **CH₂CH₂** | **CH₃ (methyl group)** | **H** |
| **(a)-2** | **H** | **CH₂CH₂** | **(CH2)₆CH₃ (heptyl group)** | **H** |
| **(a)-3** | **H** | **CH₂CH₂** | **(CH₂)₁₆CH₃ (heptadecyl group)** | **H** |
| **(a)-4** | **CH₃** | **CH₂CH₂** | **CH₃ (methyl group)** | **H** |
| **(a)-5** | **H** | **CH₂CH₂CH₂CH₂** | **CH₃ (methyl group)** | **H** |
| **(a)-6** | **H** | **CH₂CH₂** | **CH₃ (methyl group)** | **CH₃** |

**Table 2**

| **Table 2 Metal chelate complex (b) or (b')** | | | | | | | |
|---|---|---|---|---|---|---|---|
| | **M** | **R⁵** | **R⁶** | **R⁷** | **R⁸** | **g** | **h** |
| **(b)-1** | **Zr** | **CH₃** | **CH₃** | **H** | **H** | **4** | **0** |
| **(b)-2** | **Zr** | **CH₃** | **CH₃** | **H** | **H** | **1** | **3** |
| **(b)-3** | **Fe** | **CH₃** | **CH₃** | **H** | **H** | **3** | **0** |
| **(b')-1** | **Zr** | **CH₃** | **CH₃** | **H** | **H** | **0** | **4** |

**Table 3**

| **Table 3, Polymerization inhibitor (c)** | |
|---|---|
| **Polymerization inhibitor** | **Name** |
| **(c)-1** | **Tertially butyl hydroquinone** |
| **(c)-2** | **hydroquinone** |
| **(c)-3** | **Phenothiazine** |

### (Inventive examples 1 to 12 and comparative examples 1 to 8)

As shown in table 4, table 5, table 6 and table 7, the amido group-containing monomer (a), metal chelate complex (b) and polymerization inhibitor (c) were selected, blended and mixed according to the respective blending ratios to obtain the respective compositions.

Further, as the component (b`-2), zirconium isopropoxide was applied.

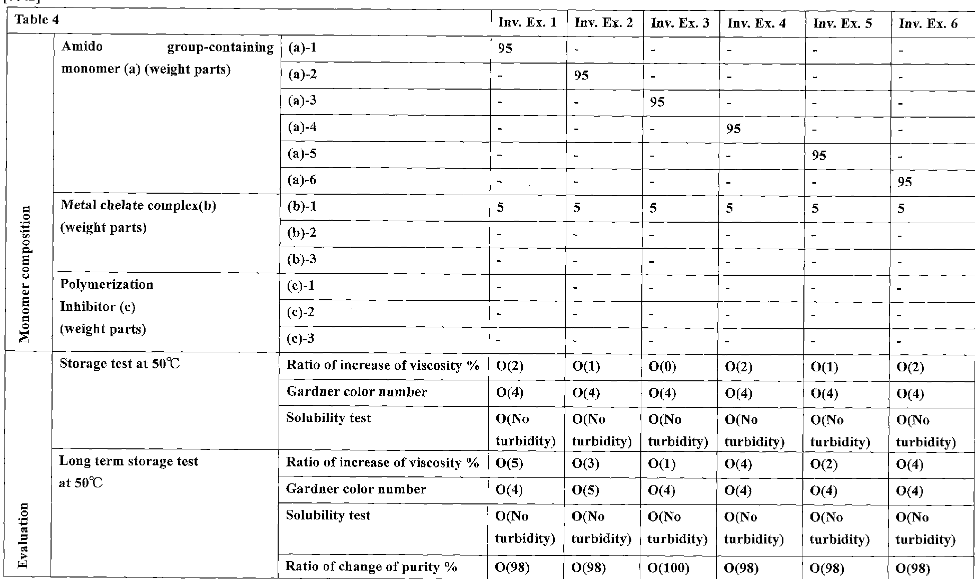

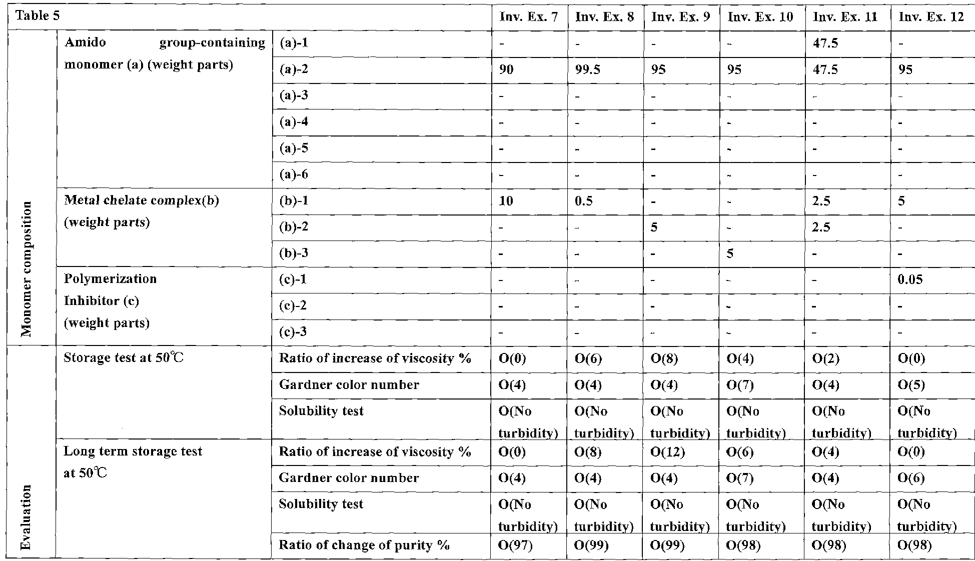

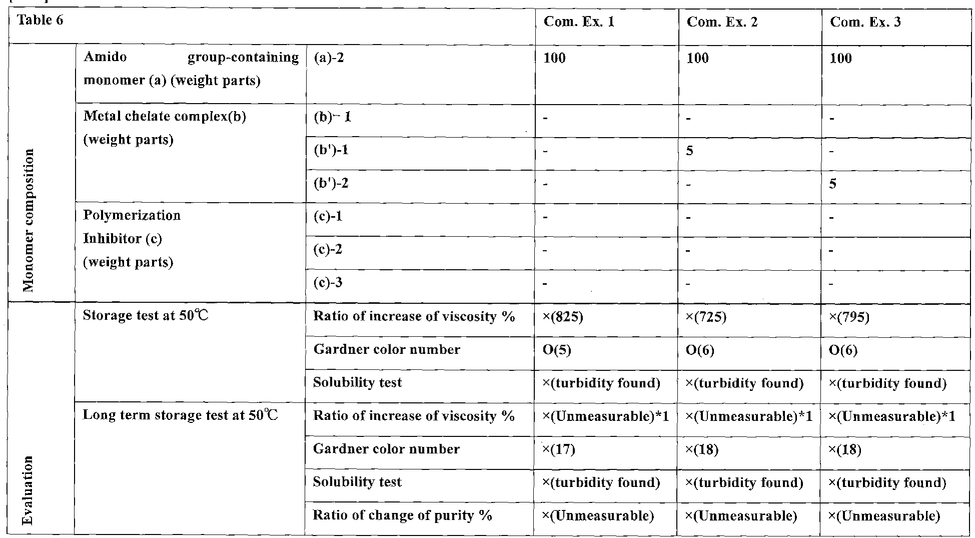

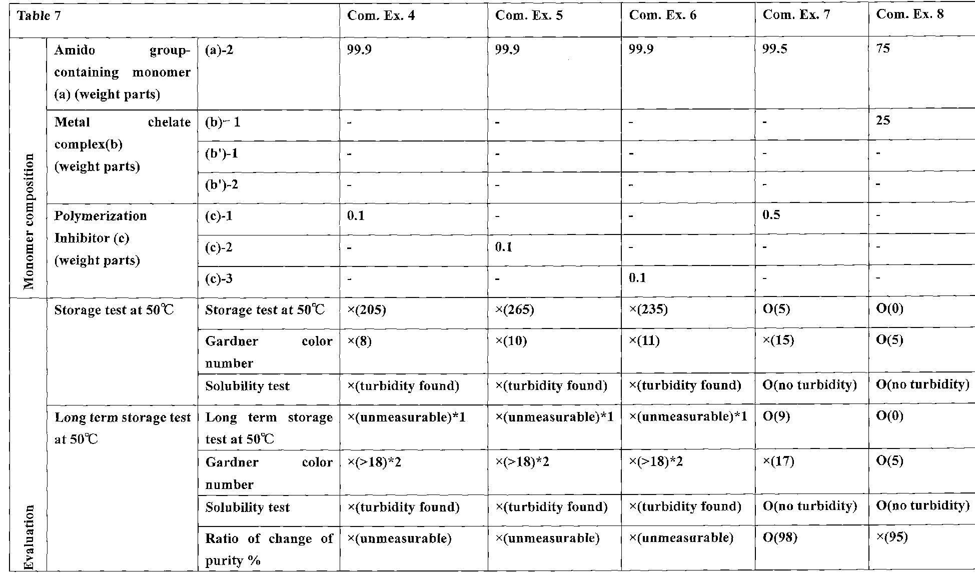

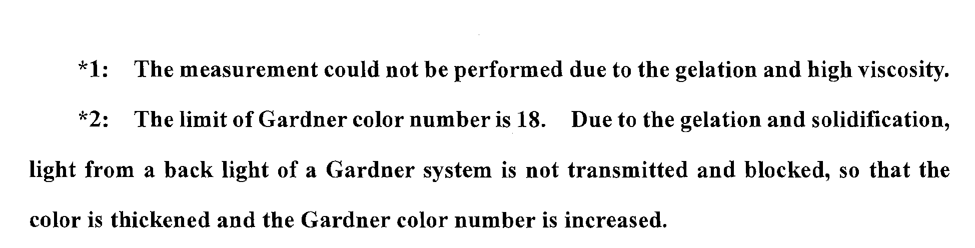

As can be seen from the results shown in table 4 and table 5, according to the inventive examples 1 to 12, the amido group-containing monomer compositions each having good ratio of increase of viscosity, Gardner color number, solubility test and ratio of change of purity were obtained. Particularly good results were obtained, in the inventive examples 1 to 8, 11 and 12 with zirconium acetylacetonate applied and in the inventive example 9 with the partial hydroxide of zirconium acetylacetonate applied.

On the other hand, according to the comparative examples 1 to 3, as the metal chelate complex (b) of the formula (2) was not added, self-polymerization occurred during the storage test at 50°C and long-term storage test at 50°C of the amido group-containing monomer composition, resulting in deterioration of the results of the ratio of increase of viscosity and solubility test.

Further, as to the comparative examples 4 to 7 in which the metal chelate complex (b) of the formula (2) was not added and instead the polymerization inhibitor was applied, self-polymerization and coloration occurred and the ratio of increase of viscosity, Gardner color number and solubility test were not good according to the comparative examples 4 to 6 in which the amount of the polymerization inhibitor was small. Further, according to the comparative example 7 in which the amount of the polymerization inhibitor is large, the coloration occurred and Gadner color number was not good.

Further, according to the comparative example 8 in which the added amount of the metal chelate complex (b) was excessive, the reduction of the purity was observed.

## Claims

1. An amido group-containing monomer composition comprising 80.00 to 99.99 mass% of an amido group-containing monomer (a) represented by a following formula (1) and 0.01 to 20.00 mass% of a metal chelate complex (b) having carbonyl group as a ligand. 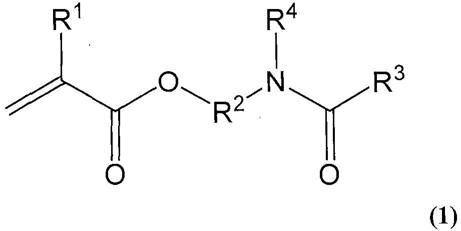 (In the formula (1),
R¹ represents hydrogen atom or methyl group,
R² represents a hydrocarbon group having a carbon number of 1 to 6,
R³ represents a hydrocarbon group having a carbon number of 1 to 23, and
R⁴ represents hydrogen or methyl group.)

2. The amido group-containing monomer composition of claim 1, wherein said metal chelate complex (b) is represented by a following formula (2). 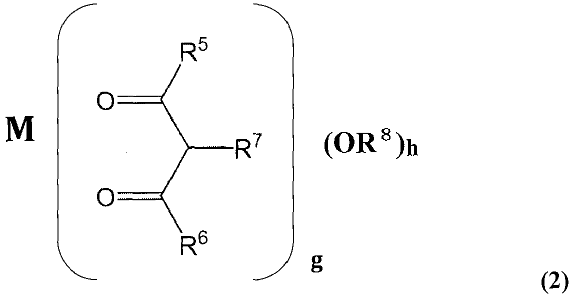 (In the formula (2),
M represents a transition metal,
g represents 1 to 4,
h represents 0 to 3,
g+h represents a total coordination number of said transition metal,
R⁵ and R⁶ independently represent a hydrocarbon group having a carbon number of 1 to 5 or phenyl group, respectively,
R⁷ represents hydrogen or a hydrocarbon group having a carbon number of 1 to 3, and
R⁸ represents hydrogen or a hydrocarbon group having a carbon number of 1 to 6.)

3. A method of improving stability of storage of an amido group-containing monomer, said method comprising adding a metal chelate complex (b) represented by a following formula (2) to an amido group-containing monomer (a) represented by a following formula (1). 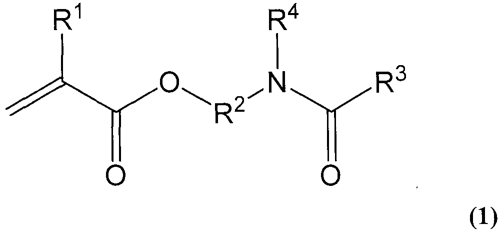 (In the formula (1),
R¹ represents hydrogen atom or methyl group,
R² represents a hydrocarbon group having a carbon number of 1 to 6,
R³ represents a hydrocarbon group having a carbon number of 1 to 23, and
R⁴ represents hydrogen or methyl group.) 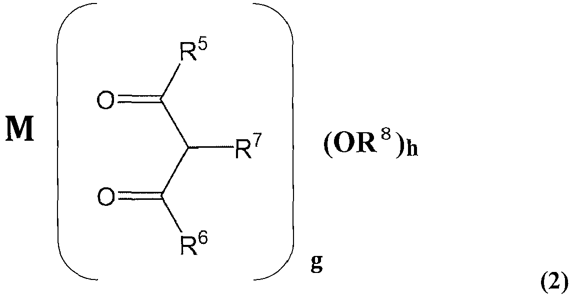 (In the formula (2),
M represents a transition metal,
g represents 1 to 4,
h represents 0 to 3,
g+h represents a total coordination number of said transition metal,
R⁵ and R⁶ independently represent a hydrocarbon group having a carbon number of 1 to 5 or phenyl group, respectively,
R⁷ represents hydrogen or a hydrocarbon group having a carbon number of 1 to 3, and
R⁸ represents hydrogen or a hydrocarbon group having a carbon number of 1 to 6.)
